# EUROPÄISCHE PATENTSCHRIFT

(11) **EP 1 602 643 B1**
(45) Veröffentlichungstag und Bekanntmachung des Hinweises auf die Patenterteilung: **19.03.2014**
(21) Anmeldenummer: 05102669.8
(22) Anmeldetag: 05.04.2005
(51) Int. Cl.: C07C 263/04, C07C 263/06, C07C 265/12

(54) **Mehrstufiges Verfahren zur kontinuierlichen Herstellung von cycloaliphatischen Diisocyanaten**
Multi-step process for the continuous preparation of cycloaliphatic diisocyanates
Procédé à plusieurs étapes pour la préparation continue de diisocyanates cycloaliphatiques

(30) Priorität: 29.05.2004 DE 102004026451
(43) Veröffentlichungstag der Anmeldung: 07.12.2005
(73) Patentinhaber: Evonik Degussa GmbH, 45128 Essen (DE)
(72) Erfinder: Kohlstruk, Stephan, 48249, Dülmen (DE); Kreczinski, Manfred, 44652 Herne (DE); Michalczak, Hans-Werner, 44651 Herne (DE)

(56) Entgegenhaltungen:
- EP-A- 0 568 782
- EP-A- 1 512 680

## Beschreibung

Die Erfindung betrifft ein mehrstufiges Verfahren zur kontinuierlichen und phosgenfreien Herstellung von cycloaliphatischen Diisocyanaten.

Der synthetische Zugang zu Isocyanaten kann über eine Reihe unterschiedlicher Routen erfolgen. Älteste und auch heute noch vorherrschende Variante zur großtechnischen Herstellung von Isocyanaten ist die so genannte Phosgenroute. Grundlage dieses Verfahrens ist Umsetzung von Aminen mit Phosgen. Nachteil des Phosgenverfahrens ist der Einsatz von Phosgen, dass aufgrund seiner Toxizität und Korrosivität besonders hohe Anforderungen an seine Handhabung im industriellen Maßstab stellt.

Es gibt mehrere Verfahren, die Verwendung von Phosgen zur Herstellung von Isocyanaten in technischen Größenordnungen zu umgehen. Der Begriff phosgenfreies Verfahren wird häufig im Zusammenhang mit der Überführung von Aminen in Isocyanate unter Einsatz alternativer Carbonylierungsmittel, z. B. Harnstoff oder Dialkylcarbonat, benutzt (EP 18 586, EP 355 443, US 4,268,683, EP 990 644).

Grundlage der so genannten Harnstoffroute ist die Harnstoff-vermittelte Überführung von Diaminen in Diisocyanate über einen zweistufigen Prozess. Im ersten Schritt wird ein Diamin mit Alkohol in Gegenwart von Harnstoff oder Harnstoff-Äquivalenten (z. B. Alkylcarbonate, Alkylcarbamate) zu einem Diurethan umgesetzt, welches üblicherweise eine Zwischenreinigungstufe durchläuft und dann im zweiten Schritt thermisch in Diisocyanat und Alkohol gespalten wird (EP 355 443, US 4,713,476, US 5,386,053). Alternativ kann der eigentlichen Urethanbildung auch die separate Herstellung eines Diharnstoffs durch gezielte Umsetzung des Diamins mit Harnstoff vorgeschaltet sein (EP 568 782). Denkbar ist auch eine zweistufige Sequenz aus partieller Umsetzung von Harnstoff mit Alkohol im ersten und anschließender Zudosierung und Urethansierung des Diamins im zweiten Schritt (EP 657 420).

Die thermische Spaltung von Urethanen in die entsprechenden Isocyanate und Alkohole ist seit langem bekannt und kann sowohl in der Gasphase bei hohen Temperaturen als auch bei relativ niedrigen Temperaturen in der Flüssigphase durchgeführt werden. Problematisch ist jedoch bei beiden Verfahrensweisen, dass durch die thermische Belastung grundsätzlich auch unerwünschte Nebenreaktionen stattfinden, die zum einen die Ausbeute mindern und zum anderen zur Bildung verharzender Nebenprodukte führen, die den Ablauf eines technischen Prozesses durch Belegungen und Verstopfungen in Reaktoren und Aufarbeitungsvorrichtungen erheblich stören.

Es hat daher nicht an Vorschlägen gefehlt, durch chemische und verfahrenstechnische Maßnahmen Ausbeuteverbesserungen zu erzielen und die unerwünschte Nebenproduktbildung einzuschränken. So wird in einer Reihe von Dokumenten der Einsatz von Katalysatoren beschrieben, die die Spaltreaktion der Urethane beschleunigen (DE 10 22 222, US 3,919,279, DE 26 35 490). Tatsächlich gelingt es in Gegenwart geeigneter Katalysatoren ― hierbei handelt es sich um eine Vielzahl basischer, saurer sowie metallorgansicher Verbindungen ― durchaus, die Isocyanatausbeute im Vergleich zur unkatalysierten Variante zu steigern. Die Bildung unerwünschter Nebenprodukte kann jedoch auch durch die Anwesenheit eines Katalysators nicht vermieden werden. Dasselbe gilt für die zusätzliche Verwendung von inerten Lösemitteln, wie sie ebenfalls in der US 3,919,279 und DE 26 35 490 empfohlen werden, um eine gleichmäßige Verteilung der zugeführten Wärme und des Katalysators im Reaktionsmedium zu gewährleisten. Grundsätzlich hat die Verwendung von unter Rückfluss siedenden Lösemitteln jedoch eine Reduzierung der Raum/Zeit-Ausbeute an Isocyanaten zur Folge und ist darüber hinaus mit dem Nachteil eines zusätzlichen hohen Energieaufwands behaftet.

In der EP 54 817 angeführte Beispiele zur thermisch geführten katalysierten Spaltung von Monourethanen beschreiben die Teilausschleusung des Reaktionsgemisches zur Abtrennung der im Zuge der Urethanspaltung entstehenden verharzenden Nebenprodukte. Diese Prozedur dient der Vermeidung von Belegungen und Verstopfungen in Reaktoren und Aufarbeitungseinrichtungen. Hinweise, die auf eine Ausbeute steigernde Verwertung der Teilausschleusung hindeuten, gibt es nicht. Die EP 061 013 beschreibt einen ähnlichen Lösungsansatz, wobei die Thermolyse in diesem Fall in Gegenwart von Lösemitteln durchgeführt wird, deren Aufgabe offenbar in einer besseren Aufnahme der schwerflüchtigen Nebenprodukte besteht. Auch hier wird die Teilausschleusung nicht im Sinne einer Ausbeuteoptimierung verwertet.

Aus der EP 0 355 443 ist nun bekannt, dass eine Ausbeutesteigerung erzielt werden kann, wenn die während der Spaltung von Diurethanen im Spaltreaktor entstehenden höhermolekularen, verwertbaren und nicht verwertbaren Nebenprodukte zur Gewährleistung einer störungsfreien und selektiven Reaktion möglichst kontinuierlich aus dem Reaktor ausgeschleust werden und anschließend in Gegenwart von Alkohol zum großen Teil umgesetzt und dann in die Diurethanherstellung zurückgeführt werden. Die beschriebene Verfahrensweise ist mit einem hohen Energieaufwand verbunden, da die Abtrennung nicht verwertbarer Nebenprodukte aus dem Austrag der Diurethanherstellung destillativ erfolgt, wobei das gesamte Diurethan verdampft werden muss. Im Unterschied zur EP 0 355 443 wird der Urethanisierungsaustrag beim Verfahren der EP 0 566 925 in zwei Teilströme aufgeteilt, von denen nur einer destillativ von seinen hochsiedenden, nicht verwertbaren Nebenprodukten befreit wird, bevor die vereinigten Diurethanströme der Deblockierungsreaktion im Spaltreaktor zugeführt werden. Zudem wird die kontinuierliche Spaltreaktorausschleusung bei der EP 566 925 direkt, d. h. ohne Reurethanisierungsschritt, in die Diurethansynthese zurückgeführt.

Die Vorgehensweise der EP 566 925 hat zur Folge, das ein Teil der Hochsiederkomponenten aus der Diurethansynthese über die Deblockierungsstufe wieder zurück in die Diurethanherstellung und weiter in die Diurethanreinigungsprozedur gelangt.

Überraschenderweise wurde gefunden, dass es bei Einsatz von cycloaliphatischen Diaminen vorteilhaft ist, die cycloaliphatischen Diurethane nach ihrer Synthese durch Umsetzung von cycloaliphatischen Diaminen mit Alkohol und Harnstoff und/oder Harnstoffderivaten von Leicht- und Mittelsiedern zu befreien, die so gereinigten cycloaliphatischen Diurethane unter Freisetzung des gewünschten cycloaliphatischen Diisocyanats thermisch zu spalten, einen Teil des Spaltsumpfes aus der Spaltapparatur kontinuierlich auszuschleusen und mit Alkohol zu reurethanisieren, die Hochsiederkomponenten abzutrennen, und den so gereinigten Stoffstrom in den Prozess zu recyclieren. Es hat sich herausgestellt, dass auf diese Weise zum einen eine vergleichsweise niedrige stationäre Konzentration an Hochsiederkomponenten über die gesamte Sequenz Diurethan-Synthese, Diurethan-Reinigung und Diurethan-Spaltung realisiert wird, so dass Ablagerungen, die insbesondere durch die von Natur aus hochviskosen Hochsiederkomponenten begünstigt werden, weitgehend vermieden werden können und auch langfristig eine gute Anlagenverfügbarkeit und eine gute Verfahrensausbeute gewährleistet sind. Zum anderen hat die der thermischen Spaltreaktion nachgeschaltete Sequenz aus Reurethanisierung und Hochsiederabtrennung den Vorteil, dass im Vergleich zur üblichen Vorgehensweise, bei der der Hochsieder vor der Diurethanspaltung abgetrennt wird, die in die Dampfphase zu überführende Diurethanmenge signifikant verringert ist, wodurch Investment- und Energiekosten eingespart werden können.

Gegenstand der Erfindung ist ein mehrstufiges Verfahren zur kontinuierlichen Herstellung von cycloaliphatischen Diisocyanaten, durch Umsetzung von cycloalipahtischen Diaminen mit Kohlensäurederivaten und Alkoholen zu cycloaliphatischen Diurethanen und anschließenden thermischen Spaltung der Diurethane zu cycloaliphatischen Diisocyanten, dadurch gekennzeichnet, dass die cycloaliphatischen Diurethane nach ihrer Synthese durch Umsetzung von cycloaliphatischen Diaminen mit Alkohol und Harnstoff und/oder Harnstoff-Derivate von Leicht- und Mittelsiedern befreit, die so gereinigten cycloaliphatischen Diurethane unter Freisetzung des gewünschten Diisocyanats thermisch gespalten, einen Teil des Spaltsumpfes aus der Spaltapparatur kontinuierlich ausgeschleust und mit Alkohol reurethanisiert wird sowie danach die Hochsiederkomponenten abgetrennt werden und der so gereinigte Stoffstrom in den Prozess recycliert wird.

Gegenstand der Erfindung ist auch ein mehrstufiges Verfahren zur kontinuierlichen Herstellung von cycloaliphatischen Diisocyanaten der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, durch Umsetzung von cycloaliphatischen Diaminen mit Harnstoff und/oder Harnstoffderivaten und Alkoholen in cycloaliphatische Diurethane und deren thermische Spaltung, wobei
a) cycloaliphatische Diamine der Formel (II)

   H₂N-R-NH₂

   wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, mit Harnstoff und/oder Harnstoffderivaten und Alkoholen der Formel (III)

   R¹-OH

   wobei R¹ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Dialkycarbonaten, Carbamidsäurealkylestern oder Mischungen aus Dialkylcarbonaten und Carbamidsäureestern und in Abwesenheit oder Gegenwart von Katalysatoren, zu cycloaliphatischen Diurethanen umgesetzt werden und man das entstehende Ammoniak gleichzeitig abtrennt;
b) aus der erhaltenen Reaktionsmischung der Alkohol, die Dialkylcarbonate und/oder Carbamidsäurealkylester abgetrennt werden und man den Alkohol sowie optional auch die Dialkylcarbonate und/oder Carbamidsäurealkylester, in die Reaktionsstufe a) zurückführt;
c) auf eine Abtrennung der in der erhaltenen Reaktionsmischung gegebenenfalls enthaltenen hochsiedenden Rückstände vollständig oder partiell verzichtet wird;
d) die über die Schritte b) und optional c) aufgereinigten Diurethane enthaltende Reaktionsmischung in Gegenwart eines Katalysators kontinuierlich und lösemittelfrei bei Temperaturen von 180 - 280 °C, vorzugsweise 200 - 260 °C, und unter einem Druck von 0,1 - 200 mbar, vorzugsweise 0,2 - 100 mbar kontinuierlich thermisch so spaltet, dass ein Teil des Reaktionsgemisches von 10 - 60 Gew.-%, bezogen auf den Feed, vorzugsweise 15 - 45 Gew.-%, bezogen auf den Feed, ständig ausgeschleust wird;
e) die Spaltprodukte durch Rektifikation in ein rohes cycloaliphatisches Diisocyanat und Alkohol getrennt werden;
f) das rohe cycloaliphatische Diisocyanat, durch Destillation gereinigt und die Reinproduktfraktion isoliert wird;
g) die Sumpfausschleusung aus d) partiell oder vollständig mit dem Alkohol aus e) in Gegenwart oder Abwesenheit von Katalysatoren innerhalb von 1 - 150 min, vorzugsweise 3 - 60 min, bei Temperaturen von 20 - 200 °C, vorzugsweise 50 - 170 °C und bei einem Druck von 0,5 - 20 bar, vorzugsweise 1 - 15 bar, umgesetzt wird, wobei das Molverhältnis von NCO-Gruppen und OH-Gruppen bis zu 1 : 100, vorzugsweise 1 : 20 und besonders bevorzugt 1 : 10 beträgt;
h) der Reurethanisatstrom aus g) in einen Wertstoff- und einen Abfallstrom aufgetrennt und der an Hochsiederkomponenten reiche Abfallstrom aus dem Prozess ausgeschleust und verworfen wird;
i) ein Teil der Sumpffraktion der Reindestillation f) kontinuierlich ausschleust und in die Spaltreaktion d) oder in die Urethanisierungsstufe g) geführt wird;
j) optional die bei der Reindestillation f) des rohen cycloaliphatischen Diisocyanats anfallende Kopffraktion ebenfalls in die Urethanisierungsstufe g) zurückgeführt wird;
k) der Wertstoffstrom aus h) in Stufe a) ,b) oder d) zurückgeführt wird.

Nach dem erfindungsgemäßen Verfahren können cycloaliphatische Diisocyanate im kontinuierlichen Betrieb problemlos mit sehr guten Ausbeuten hergestellt werden. Vorteilhaft bei dem erfindungsgemäßen Mehrstufenverfahren ist insbesondere die Tatsache, dass auch bei Einsatz von cycloaliphatischen Diaminen der Formel (II) als Ausgangsmaterial für die kontinuierliche Diisocyanatsynthese Ablagerungen, die insbesondere durch die von Natur aus hochviskosen Hochsiederkomponenten begünstigt werden, weitgehend vermieden werden können und auch langfristig eine gute Anlagenverfügbarkeit und eine gute Verfahrensausbeute gewährleistet sind. Weiterhin ist es ein Vorteil des erfindungsgemäßen Mehrstufenverfahrens, dass es erlaubt, die Menge des in die Dampfphase zu überführenden Diurethans auf ein Minimum zu verringern und auf diese Weise den notwendigen Energieaufwand beschränkt.
a) Zur Herstellung der monomeren cycloaliphatischen Diurethane in der Reaktionsstufe a) werden die cycloaliphatischen Diamine der Formel (II) mit Harnstoff und/oder Harnstoffderivaten und einem Alkohol der Formel (III), gegebenenfalls auch Mischungen solcher Alkohole, in einem Molverhältnis von 1 : 2,01 : 4,0 bis 1 : 2,2 : 10, vorzugsweise 1 : 2,02 : 6 bis 1 : 2,12 : 9, gegebenenfalls aber nicht vorzugsweise in Gegenwart von Dialkycarbonaten, Carbamidsäurealkylestern oder Mischungen aus Dialkylcarbonaten und Carbamidsäureestern in einer Menge von jeweils 1 - 10 Mol-% bezogen auf das das Diamin, in Abwesenheit oder Gegenwart von Katalysatoren bei Reaktionstemperaturen von 140 - 270 °C, vorzugsweise 160 - 250 °C und unter einem Druck, der in Abhängigkeit vom eingesetzten Alkohol zwischen 2 - 80 bar, vorzugsweise 7 - 15 bar beträgt, innerhalb von 2 bis 20 Stunden, vorzugsweise 4 - 9 Stunden, zur Reaktion gebracht. Die Umsetzung kann in einer kontinuierlich betriebenen Rührkesselkaskade, vorzugsweise aber in einem Druckdestillationsreaktor, erfolgen.

Zur Erhöhung der Reaktionsgeschwindigkeit können die cycloaliphatischen Diurethane, in Gegenwart von Katalysatoren hergestellt werden. Als Katalysatoren eignen sich anorganische oder organische Verbindungen, die ein oder mehrere, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14^{th} Edition, publiziert von Chemical Rubber Publishing Co. 2310 Superior Ave. N.E. Cleveland, Ohio, enthalten, beispielsweise Halogenide wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocaramate. Beispielhaft genannt seinen die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen, Kobalt und Nickel. Als typische Katalysatoren seinen beispielhaft folgende Verbindungen genannt: Lithiumethanolat, Lithiumbutanolat, Natriummethanolat, Kalium-tert.-butanolat, Magnesiumethanolat, Calciummethanolat, Zinn-(II)-chlorid, Zinn-(IV)-chlorid, Bleiacetat, Aluminiumtrichlorid, Bismuttrichlorid, Kupfer-(II)-acetat, Kupfer-(II)-chlorid, Zinkchlorid, Zinkoctoat, Titantetrabutanolat, Vanadiumtrichlorid, Vanadiumacetylacetonat, Mangan-(II)-acetat, Eisen-(II)-acetat, Eisen-(III)-acetat, Eisenoxalat, Cobaltchlorid, Cobaltnaphthenat, Nickelchlorid, Nickelnaphthenat sowie deren Mischungen. Die Katalysatoren können gegebenenfalls auch in Form ihrer Hydrate oder Ammoniakate zu Einsatz kommen.

Ausgangsverbindungen für das erfindungsgemäße Verfahren sind cycloaliphatische Diamine der bereits obengenannten Formel (II), Alkohole der bereits obengenannten Formel (III) sowie Harnstoff und/oder als Carboxylierungsmittel geeignete Harnstoffderivate (Kohlensäurederivate) in Abwesenheit oder Gegenwart von Dialkylcarbonaten, Carbamidsäurealkylestern oder Mischungen aus Dialkylcarbonaten und Carbamidsäurealkylestern.

Geeignete Diamine der Formel (II) sind beispielsweise 1,4-Diaminocyclohexan, 4,4'-Methylendicyclohexyldiamin, 2,4-Methylendicyclohexyldiamin, 2,2'-Methylendicyclohexyldiamin und isomere cycloaliphatische Diamine sowie perhydriertes Methylendiphenyldiamin. Methylendiphenyldiamin (MDA) fällt herstellungsbedingt als Isomerenmischung aus 4,4'-, 2,4- und 2,2'-MDA an (s. z. B. DE 101 27 273). Perhydriertes Methylendiphenyldiamin wird durch vollständige Hydrierung von MDA erhalten und ist demzufolge eine Mischung aus isomeren Methylendicyclohexyldiaminen (H₁₂MDA), nämlich 4,4'-, 2,4- und 2,2'-H₁₂MDA und eventuell geringen Mengen an nicht vollständig umgesetzten (teil)aromatischen MDA. Bevorzugt werden als Diamine der Formel (II) 4,4'-Methylendicyclohexyldiamin, 2,4-Methylendicyclohexyldiamin und 2,2'-Methylendicyclohexyldiamin sowie auch beliebige Mischungen mindestens zweier dieser Isomere eingesetzt. Selbstverständlich können auch Diamine zum Einsatz gelangen, die von der Formel (II) abweichen. Beispielhaft seinen 1,3- und 1,4-Diaminomethylcyclohexan, Hexandiamin-1,6, 2,2,4- bzw. 2,4,4-Trimethylhexanamin-1,6 und 3-Aminomethyl-3,5,5-trimethylcyclohexyl-amin aufgeführt. Der Einsatz von Aminen, die von der Formel (II) abweichen, ist jedoch nicht bevorzugt.

Als Alkohole der Formel (III) eignen sich beliebige aliphatische oder cycloaliphatische Alkohole, die unter Normaldruck einen unterhalb 190°C liegenden Siedepunkt aufweisen. Beispielhaft genannt seien C1-C6-Alkanole wie z. B. Methanol, Ethanol, 1-Propanol, 1-Butanol, 2-Butanol, 1-Hexanol oder Cyclohexanol. Bevorzugt wird 1-Butanol als Alkohol verwendet.

Im Zuge der Umsetzung des Reaktionsgemisches wird Ammoniak freigesetzt, dessen Entfernung aus dem Reaktionsgleichgewicht sich als vorteilhaft erwiesen hat. Beim Austrag des Ammoniaks aus dem Reaktor ist darauf zu achten, dass die Wandtemperaturen des Reaktors und des Austragsrohres oberhalb von 60°C liegen, damit eine Belegung durch Ammoniumcarbaminat, welches in minimalen Mengen aus Ammoniak und Kohlendioxid durch Zersetzung von Harnstoff gebildet wird, vermieden werden kann. Es hat sich beispielsweise bewährt, die Umsetzung in einem Druckdestillationsreaktor durchzuführen, wobei das Reaktionsgemisch im Gegenstrom zu im Sumpf eingebrachten Alkoholbrüden geführt wird und auf diese Weise eine derartig intensive Durchmischung der Flüssigkeit auf den Böden, dass die praktisch jeweils einer Kaskadenstufe entsprechen, erfolgt. Das am Kopf abgezogene, dampfförmige Gemisch aus Alkohol und Ammoniak kann, vorzugsweise unter dem Druck des Druckdestillationsreaktors und ohne es vorher zu kondensieren, in eine Destillationskolonne geführt werden, vom Ammoniak freien Alkohol zu gewinnen, der in den Sumpf des Druckdestillationsreaktors und der Kolonne zurückgeführt wird. Um eine Belegung des Rückflußkondensators mit Ammoniumcarbaminat zu verhindern, lässt man in diesem zur Einstellung der Temperatur am Kopf auf mindestens 60 °C einen entsprechenden Anteil an Alkohol zu.

b) Der überschüssige Alkohol, die Dialkylcarbonate, sofern solche gebildet wurden oder in der Reaktionsmischung vorliegen, oder Carbamidsäurealkylester oder Mischungen aus mindestens zwei dieser Komponenten werden vorteilhafterweise zweistufig abgetrennt. Auf der ersten Stufe wird die Reaktionsmischung vom Druckniveau der Reaktionsstufe a) auf einen Druck von 1 - 500 mbar, vorzugsweise 2 - 150 mbar, entspannt und auf diese Weise in gasförmige Brüden, die die überwiegende Alkoholmenge sowie gegebenenfalls Dialkylcarbonate und/oder Carbamidsäurealkylester enthalten, und in einen flüssigen Austrag aufgetrennt. Im zweiten Schritt wird der flüssige Austrag durch Dünnschichtverdampfung bei 180 - 250 °C, bevorzugt 200 - 230 °C, und einem Druck von 0,1 - 20 mbar, vorzugsweise 1 - 10 mbar, von gegebenenfalls vorhandenem restlichen Alkohol sowie Mittelsiedern wie Dialkylcarbonaten und/oder Carbwnidsäurealkylestem befreit, so dass der Rückstand im wesentlichen aus dem monomeren Polyurethan, vorzugsweise Diurethan, und gegebenenfalls hochsiedenden Oligomeren besteht.

Die Brüden können, vorzugsweise nach destillativer Reinigung, optional in die Reaktionsstufe a) zurückgeführt werden.
c) Bevorzugt wird auf jedwede Abtrennung der in der Reaktionsmischung aus Stufe b) gegebenenfalls enthaltenen Hochsieder verzichtet. Sofern die unter h) beschriebene Auftrennung des Reurethanisatstroms aus Stufe g) jedoch nur mit einem Teilstrom, d. h. partiell, durchgeführt wird, kann es vorteilhaft sein, die anschließend erläuterten Wege zur Hochsiederabtrennung zu beschreiten:

Optional kann der nach Abtrennung von Leicht- und Mittelsiedem erhaltene flüssige, die monomeren Diurethane und gegebenenfalls hochsiedende Oligomere enthaltende Stoffstrom aus Schritt b), vorzugsweise mit Hilfe eines Dünnschicht- oder Kurzwegverdampfers, bei einer Temperatur von 180 - 270 °C, vorzugsweise 200 - 250 °C und unter einem Druck von 0,01 - 10 mbar, vorzugsweise 0,02 - 5 mbar destillativ in einen Wertstoffstrom, der die monomeren Diurethane und die leichter siedenden Nebenprodukte enthält, und einen nicht destillierbaren Nebenproduktstrom getrennt werden. Der die hochsiedenden Komponenten enthaltende, nicht destillierbare Nebenproduktstrom wird aus dem Herstellverfahren ausgeschleust und üblicherweise als stofflich nicht verwertbarer Rückstand verworfen.
Optional kann der gegebenenfalls hochsiedende Oligomere enthaltende Stoffstrom aus Stufe b) vor seiner oben beschriebenen destillativen Aufreinigung auch in zwei Teilströme aufgeteilt werden, von denen einer direkt der Deblockierungsreaktion (siehe d)) zugeführt wird und der andere zunächst die oben beschriebene Hochsiederabtrennung durchläuft.
d) Der die monomeren Diurethane und die leichter siedenden Nebenprodukte enthaltende Wertstoffstrom aus Stufe b) und optional aus Stufe c) wird in einer geeigneten Vorrichtung teilweise, lösemittelfrei in flüssiger Phase in Gegenwart von Katalysatoren bei Temperaturen von 180 - 280 °C, vorzugsweise 200 - 260 °C, und unter einem Druck von 0,1 - 200 mbar, vorzugsweise 0,2 - 100 mbar kontinuierlich thermisch gespalten. Der Umsatz von Diurethan zu Diisocyanat in der Vorrichtung zur thermischen Spaltung kann in Abhängigkeit vom verwendeten Diurethan weitgehend frei gewählt werden und liegt üblicherweise in einem Bereich von 10 - 95 Gew.-%, vorzugsweise 35 - 85 Gew.-% der zugeführten Diurethanmenge (Feed). Der ungespaltende Anteil der Reaktionsmischung, der nicht umgesetzte Diurethane, hochsiedende Nebenprodukte und andere wieder verwertbare und unverwertbare Nebenprodukte enthält, wird kontinuierlich ausgeschleust. Die Menge der Ausschleusung richtet sich u.a. nach dem gewünschten Umsatz und der gewünschten Kapazität der Spaltreaktion und kann leicht experimentell ermittelt werden. Sie beträgt üblicherweise 10 - 60 Gew.-%, vorzugsweise 15 - 45 Gew.-%, bezogen auf den Feed.
   Als Katalysatoren zur chemischen Spaltung der Diurethane finden z. B. die vorgenannten, die Urethanbildung katalysierenden anorganischen und organischen Verbindungen Verwendung. Vorzugsweise werden Chloride des Zinks oder Zinns sowie Zink-, Mangan-, Eisen-, oder Cobaltoxide eingesetzt, wobei der Katalysator dem im wesentlichen Diurethane enthaltenden Stoffstrom aus der Reinigungssequenz b) und optional c) vor dessen Zuführung in die Spaltung als 0,01 -25 Gew.-%ige, vorzugsweise 0,05 - 10 Gew.-%ige Lösung oder Suspension vorzugsweise in dem Alkohol, der auch zur Urethanherstellung verwendet wird, in einer Menge von 5 - 400 ppm, vorzugsweise 10 - 100 ppm, zudosiert wird.
   Als Spaltvorrichtungen eigenen sich beispielsweise zylinderförmige Spaltreaktoren, wie z. B. Röhrenöfen oder vorzugsweise Verdampfer, beispielsweise Fallfilm-, Dünnschicht- oder Bulkverdampfer, wie z. B. Robertverdampfer, Herbertverdampfer, caddle-typ-Verdampfer, Oskarverdampfer und Heizkerzenverdampfer.
   Prinzipiell geht es darum, die mittlere Verweilzeit der Isocyanatgruppen, die bei Deblockierung des Alkohols zwangsläufig freigesetzt werden, in der Spaltzone möglichst gering zu halten und so unerwünschte Nebenreaktionen auf ein Minimum zu beschränken.
   Bevorzugt wird die Spaltung in einer kombinierten Spalt- und Rektifizierkolonne durchgeführt, die für die Energiezufuhr im Sumpf mit einem Fallfilmverdampfer, im unteren Drittel mit einer Einrichtung zum zusätzlichen Energieeintrag bzw. zur Energierückgewinnung, im oberen Drittel mit einer Einrichtung zum Abzug von Roh-Diisocyanat und am Kopf mit einem Kondensator für den Rückfluss und den Abzug von reinem Alkohol ausgestattet ist.
e) Die bei der thermischen Spaltung gebildeten Spaltprodukte, die sich vor allem aus Alkohol, Diisocyanat und partiell gespaltenen Diurethanen zusammensetzen, werden durch Rektifikation bei Temperaturen von 95 - 260 °C, vorzugsweise 110 - 245 °C und einem Druck von 0,5 - 250 mbar, vorzugsweise 1 - 100 mbar, in Alkohol und in eine rohe Diisocyanatmischung - bevorzugt bestehend aus cycloaliphatischem Diisocyanat, partiell gespaltenem cycloaliphatischem Diisocyanat und gegebenenfalls geringen Anteilen an cycloaliphatischen Diurethan ― getrennt. Diese Trennung kann beispielsweise in der Spaltkolonne der obengenannten kombinierten Spalt- und Rektifizierkolonne durchgeführt werden.
f) Die vorzugsweise durch Rektifikation erhaltene rohe Mischung, bestehend aus cycloaliphatischem Diisocyanat, partiell gespaltenem cycloaliphatischem Diurethan und gegebenenfalls geringen Anteilen an cycloaliphatischen Diurethan, wird durch Destillation bei einer Temperatur von 95 - 260 °C, vorzugsweise 110 - 245°C und unter einem Druck von 0,5 - 150 mbar, vorzugsweise 1 - 75 mbar, gereinigt, wobei die anfallenden Fraktionen zurückgeführt oder als Reinprodukt isoliert werden.
g) Die Sumpfausschleusung aus der Spaltstufe d) wird partiell oder vollständig mit dem Alkohol aus der Rektifikationsstufe e) zusammengeführt, wobei das Molverhältnis von NCO-Gruppen und OH-Gruppen bis zu 1 : 100, vorzugsweise 1 : 20 und besonders bevorzugt 1 : 10 beträgt, und die Reaktionsmischung in Gegenwart oder Abwesenheit von Katalysatoren innerhalb von 1 -150 min, vorzugsweise 3 - 60 min, bei Temperaturen von 20 - 200 °C, vorzugsweise 50 -170 °C und bei einem Druck von 0,5 - 20 bar, vorzugsweise 1 - 15 bar, umgesetzt. Die Umsetzung kann in einer kontinuierlichen Kesselkaskade oder in einem Rohrreaktor durchgeführt werden. Als Katalysatoren kommen grundsätzlich alle Kontakte in Frage, die die NCO/OH-Reaktion fördern. Beispielhaft seien Zinnoctoat, Dibutylzinnlaurat, Zinndichlorid, Zinkdichlorid und Triethylamin aufgeführt. Die Reurethanisierung kann auch in Gegenwart von Fe(III)- oder Cu(I)-Halogeniden oder Mischungen davon durchgeführt werden. Beispielhaft seinen Fe(III)-chlorid, Fe(III)-bromid, Cu(I)-chlorid und Cu(I)-bromid aufgeführt. Der Einsatz dieser Katalysatoren schließt die gleichzeitige Verwendung anderer Katalysatoren, die der Beschleunigung der Urethanisierung dienen, nicht grundsätzlich aus. Bevorzugt werden die Halogenide von Fe(III) oder Cu(I) oder Mischungen davon ohne zusätzliche Verwendung weiterer Kontakte eingesetzt.
h) Der Reurethanisatstrom aus Stufe g) wird in einen Wertstoff- und einen Abfallstrom aufgetrennt und der an Hochsiederkomponenten reiche Abfallstrom aus dem Prozess ausgeschleust und verworfen. Die Auftrennung der beiden Stoffströme erfolgt vorzugsweise destillativ mit Hilfe eines Dünnschicht- oder Kurzwegverdampfers, bei einer Temperatur von 180 - 270 °C, vorzugsweise 200 - 250 °C und unter einem Druck von 0,01 -10 mbar, vorzugsweise 0,02 - 5 mbar. Der Wertstoffstrom, der die monomeren Diurethane und die leichter siedenden Nebenprodukte enthält, fällt als Destillat an. Der an hochsiedenden Komponenten reiche Abfallstrom fällt als Rückstand an und wird aus dem Herstellverfahren ausgeschleust und üblicherweise als stofflich nicht verwertbareres Material verworfen. Alternativ, aber nicht bevorzugt, kann die Auftrennung in Wert- und Abfallstoff auch durch Extraktion erfolgen. Als Extraktionsmittel ist beispielsweise überkritisches Kohlendioxid geeignet.
   Optional kann der Reurethanisatstrom vor der oben beschriebenen Aufreinigung auch in zwei Teilströme aufgeteilt werden, von denen einer direkt der Reinigungsstufe b) zugeführt wird. Die Aufteilung der beiden Teilströme kann im Verhältnis 99 : 1 bis 1 : 99, bevorzugt 95 : 5 bis 5 : 95 erfolgen. Optional kann der zur Hochsiederabtrennung führende Reurethanisatstrom zunächst partiell oder vollständig von überschüssigem Alkohol befreit werden. Dies erfolgt vorzugsweise destillativ. Der abgetrennte Alkohol kann wahlweise zur Stufe a) oder b) zurückgeführt werden.
i) Ein Teil der Sumpffraktion der Reindestillation f) wird kontinuierlich ausgeschleust und optional in die Spaltstufe d) oder in die Urethanisierungsstufe g) zurückgeführt. Die Rückführung in die Urethanisierungsstufe ist bevorzugt. Die Menge der Ausschleusung beträgt 0,1 - 50 Gew.-%, vorzugsweise 0,2 - 25 Gew.-% des Zulaufs an rohem Polyisocyanat in die Reindestillationsstufe.
j) Die Kopffraktion der Reindestillationstufe f) kann vorworfen oder vorzugsweise in die Urethanisierungsstufe g) zurückgeführt werden. Die Menge der pro Zeiteinheit abgeführten Kopffraktion beträgt 0,1-3 Gew.-%, vorzugsweise 0,3-1 Gew.-% des Zulaufs an rohem Polyisocyanat in die Reindestillation.
k) Der Wertstoffstrom aus Stufe h) wird in die Diurethanherstellung a), die Leicht- und Mittelsiederabtrennung b) oder die Diurethanspaltung d) zurückgeführt. Eine Rückführung in Stufe c) ist ebenfalls möglich, aber nicht bevorzugt.

Mit dem erfindungsgemäßen mehrstufigen Verfahren zur kontinuierlichen Herstellung von cycloaliphatischen Diisocyanaten unter Rückführung und Ausschleusung der Nebenprodukte kann für destillierbare cycloaliphatische Diisocyanate über einen langen Zeitraum eine mit hoher Selektivität störungsfrei ablaufende Reaktion gewährleistet werden. Das erfindungsgemäße Verfahren eignet sich insbesondere zur Herstellung von cycloaliphatischen Diisocyanaten mit 4 bis 18, vorzugsweise 5 bis 15 Kohlenstoffatomen wie 1,4-Diisocyantocyclohexan, 4,4'-Methylendicyclohexyldiisocyanat (4,4'-H₁₂MDI), 2,2'-Methylendicyclohexyldiisocyanat (2,2'-H₁₂MDI), 2,4-Methylendicyclohexyldiisocyanat (2,4-H₁₂MDI) oder auch Mischungen der vorgenannten isomeren Methylendicyclohexyldiisocyanate, wie sie zum Beispiel naturgemäß bei der Umwandlung von perhydriertem MDA in H₁₂MDI anfallen.

Die hergestellten cycloaliphatischen Diisocyanate, eigenen sich bestens zur Herstellung von Urethan-, Isocyanurat-, Amid- und/oder Harnstoffgruppen enthaltenden Kunststoffen nach dem Polyisocyanat-Polyadditionsverfahren. Sie finden darüber hinaus Verwendung zur Herstellung von mit Urethan-, Biuret-, und/oder Isocyanuratgruppen modifizierten Polyisocyanatmischungen. Derartige Polyisocyanatmischungen aus cycloaliphatischen Diisocyanaten werden insbesondere zur Herstellung von hochwertigen, lichtbeständigen Polyurethanbeschichtungen eingesetzt.

Die Erfindung wird durch folgendes Beispiel näher erläutert.

### Beispiel

**Beispiel:** Erfindungsgemäße Herstellung von Methylendicyclohexyldiisocyanat (H₁₂MDI) aus perhydriertem Methylendiphenyldiamin (H₁₂MDA) und Harnstoff in Gegenwart von n-Butanol.

Der oberste Boden eines Druckdestillationsreaktors wurden stündlich mit 281,5 g H₁₂MDA, 164,1 g Harnstoff und 599,7 g n-Butanol beaufschlagt und die Reaktionsmischung unter kontinuierlicher Entfernung des freigesetzten Ammoniaks bei 11 - 14 bar, 220 °C und einer mittleren Verweilzeit von 8,5 h gekocht. Der Reaktoraustrag wurde, zusammen mit dem Stoffstrom aus der Hochsiederabtrennung, im Flash-Behälter bei 55 mbar und anschließender Dünnschichtverdampfung bei 220 °C und 2 mbar von überschüssigem Alkohol, Leicht- und Mittelsiedern befreit und die verbleibenden 780,5 g/h Bis-(4-butoxycarbonylaminocyclohexyl)-methan (H₁₂MDU) als Schmelze (140 °C) in die Wälzung des Fallfilmverdampfers der Spalt- und Rektifizierkolonne gefahren, wobei die Deblockierungsreaktion bei einer Temperatur von 232 °C und einem Sumpfdruck von 9 mbar in Gegenwart einer stationären Konzentration an Zinndichlorid von 20 ppm durchgeführt wurde. Die Spaltgase H₁₂MDI und Butanol wurden in zwei hintereinandergeschalteten Kondensatoren bei 85 °C und -25 °C auskondensiert. Das gewonnene, etwa 97 %ige Roh- H₁₂MDI wurde einer Reindestillation zugeführt, wobei 321,3 g/h H₁₂MDI mit einer Reinheit von > 99,5 % erhalten wurden, was einer Ausbeute von 92 % entspricht. 228,1 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurde kontinuierlich ein Teilstrom aus dem Wälzkreislauf ausgeschleust und zusammen mit 23,9 g/h Sumpfauskreisung aus der H₁₂MDI-Reindestillation sowie dem Kopfprodukt aus der Spalt- und Rektifizierkolonne vereinigt und reurethanisiert. Der Reurethanisatstrom wurde durch Flash-Verdampfung bei 40 mbar von überschüssigem Butanol befreit und mittels eines Kurzwegverdampfers bei 235 °C und einem Druck von 0,04 mbar in einen Hochsieder reichen Abfallstrom und einen Wertstoffstrom aufgetrennt. Die 231,1 g/h Wertstoffstrom wurden zusammen mit dem Reaktoraustrag der Diurethanherstellung dem Flash-Behälter zugeführt.

## Patentansprüche

1. Mehrstufiges Verfahren zur kontinuierlichen Herstellung von cycloaliphatischen Diisocyanaten, durch Umsetzung von cycloalipahtischen Diaminen mit Kohlensäurederivaten und Alkoholen zu cycloaliphatischen Diurethanen und anschließenden thermischen Spaltung der Diurethane zu cycloaliphatischen Diisocyanaten,
**dadurch gekennzeichnet,**
**dass** die cycloaliphatischen Diurethane nach ihrer Synthese durch Umsetzung von cycloaliphatischen Diaminen mit Alkohol und Harnstoff und/oder Harnstoff-Derivate von Leicht- und Mittelsiedern befreit, die so gereinigten cycloaliphatischen Diurethane unter Freisetzung des gewünschten Diisocyanats thermisch gespalten, einen Teil des Spaltsumpfs aus der Spaltapparatur kontinuierlich ausgeschleust und mit Alkohol reurethanisiert wird sowie danach die Hochsiederkomponenten abgetrennt werden und der so gereinigte Stoffstrom in den Prozess recycliert wird.

2. Mehrstufiges Verfahren nach Anspruch 1 zur kontinuierlichen Herstellung von cycloaliphatischen Diisocyanaten der Formel (I)
OCN-R-NCO
wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, durch Umsetzung von cycloaliphatischen Diaminen mit Harnstoff und/oder Harnstoffderivaten und Alkoholen in cyclialiphatische Diurethane und deren thermische Spaltung, wobei
a) cycloaliphatische Diamine der Formel (II)
H₂N-R-NH₂
wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, mit Harnstoff und/oder Harnstoffderivaten und Alkoholen der Formel (III)
R¹-OH
wobei R¹ für einen Rest steht, wie er nach Entfernung der Hydroxylgruppe aus einem primären oder sekundären (cyclo)aliphatischen Alkohol mit 3 bis 8 Kohlenstoffatomen verbleibt,
in Abwesenheit oder Gegenwart von Dialkycarbonaten, Carbamidsäurealkylestern oder Mischungen aus Dialkylcarbonaten und Carbamidsäureestern und in Abwesenheit oder Gegenwart von Katalysatoren, zu cycloaliphatischen Diurethanen umgesetzt werden und man das entstehende Ammoniak gleichzeitig abtrennt;
b) aus der erhaltenen Reaktionsmischung der Alkohol, die Dialkylcarbonate und/oder Carbamidsäurealkylester abgetrennt werden und man den Alkohol sowie optional auch die Dialkylcarbonate und/oder Carbamidsäurealkylester, in die Reaktionsstufe a) zurückführt;
c) auf eine Abtrennung der in der erhaltenen Reaktionsmischung gegebenenfalls enthaltenen hochsiedenden Rückstände vollständig oder partiell-verzichtet wird;
d) die über die Schritte b) und optional c) aufgereinigten Diurethane enthaltende Reaktionsmischung in Gegenwart eines Katalysators kontinuierlich und lösemittelfrei bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch so spaltet, dass ein Teil des Reaktionsgemisches von 10 bis 60 Gew.-%, bezogen auf den Feed, vorzugsweise 15 bis 45 Gew.-%, bezogen auf den Feed, ständig ausgeschleust wird;
e) die Spaltprodukte durch Rektifikation in ein rohes cycloaliphatisches Diisocyanat und Alkohol getrennt werden;
f) das rohe cycloaliphatische Diisocyanat durch Destillation gereinigt und die Reinproduktfraktion isoliert wird;
g) die Sumpfäusschleusung aus d) partiell oder vollständig mit dem Alkohol aus e) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird, wobei das Molverhältnis von NCO-Gruppen und OH-Gruppen bis zu 1 : 100, vorzugsweise 1 : 20 und besonders bevorzugt 1 : 10 beträgt;
h) der Reurethanisatstrom aus g) in einen Werstoffstrom- und einen Abfallstrom aufgetrennt und der an Hochsiederkomponenten reiche Abfallstrom aus dem Prozess ausgeschleust und verworfen wird;
i) ein Teil der Sumpffraktion der Reindestillation f) kontinuierlich ausschleust und in die Spaltreaktion d) oder in die Urethanisierungsstufe g) geführt wird;
j) optional die bei der Reindestillation f) des rohen cycloaliphatischen Diisocyanats anfallende Kopffraktion ebenfalls in die Urethanisierungsstufe g) zurückgeführt wird;
k) der Werstoffstrom aus h) in Stufe a), b) oder d) zurückgeführt wird.

3. Mehrstufiges Verfahren nach Anspruch 1 oder 2,
**dadurch gekennzeichnet,**
**dass** als cycloaliphatisches Diamin 4,4'-Methylendicyclohexyldiamin, 2,4-Methylendicyclohexyldiamin und 2,2'-Methylendicyclohexyldiamin sowie auch beliebige Mischungen mindestens zweier dieser Isomere eingesetzt werden.

4. Mehrstufiges Verfahren nach Anspruch 1 oder 2,
**dadurch gekennzeichnet,**
**dass** als cycloaliphatisches Diamin 4,4'-Methylendicyclohexyldiamin und/oder isomere cycloaliphatische Diamine eingesetzt werden.

5. Mehrstufiges Verfahren nach Anspruch 1 oder 2,
**dadurch gekennzeichnet,**
**dass** als cycloaliphatisches Diamin 1,4-Diaminocyclohexan eingesetzt wird.

6. Mehrstufiges Verfahren nach mindestens einem der vorherigen Ansprüche,
**dadurch gekennzeichnet,**
**dass** die Stufe a) kontinuierlich in einem Destillationsreaktor oder in einer Rührkesselkaskade durchgeführt wird.

7. Mehrstufiges Verfahren nach mindestens einem der vorherigen Ansprüche,
**dadurch gekennzeichnet,**
**dass** die Umsetzung in Stufe a) in einem Molverhältnis von Diamin : Harnstoff : Alkohol von 1 : 2,01 : 4,0 bis 1 : 2, 2 : 10 erfolgt.

8. Mehrstufiges Verfahren nach mindestens einem der vorherigen Ansprüche,
wobei die Verweilzeit der Edukte in Stufe a) 2 bis 20, vorzugsweise 4 bis 9 Stunden beträgt.

9. Mehrstufiges Verfahren nach mindestens einem der vorherigen Ansprüche,
**dadurch gekennzeichnet,**
**dass** die Stufe a) in einem Reaktor bei 140 bis 270 °C und einem Druck von 2 bis 80 bar durchgeführt wird.

10. Mehrstufiges Verfahren nach mindestens einem der vorherigen Ansprüche,
**dadurch gekennzeichnet,**
**dass** in Stufe a) bei Reaktionstemperaturen von 160 bis 250 °C und bei einem Druck von 7 bis 15 bar umgesetzt wird.

11. Mehrstufiges Verfahren nach mindestens einem der vorherigen Ansprüche,
**dadurch gekennzeichnet,**
**dass** die Stufe a) in einem Druckdestillationsreaktor durchgeführt wird.

12. Mehrstufiges Verfahren nach mindestens einem der vorherigen Ansprüche,
wobei in Stufe a) die Edukte kontinuierlich auf den obersten Boden aufgegeben werden und das freigesetzte Ammoniak unterstützt durch Alkoholbrüden, die im Sumpf des Destillationsreaktor eingeführt werden, ausgetrieben wird.

13. Mehrstufiges Verfahren nach mindestens einem der vorherigen Ansprüche,
**dadurch gekennzeichnet,**
**dass** in Stufe a) Alkohole mit 1 - 6 Kohlenstoffatomen eingesetzt werden.

14. Mehrstufiges Verfahren nach mindestens einem der vorherigen Ansprüche,
**dadurch gekennzeichnet,**
**dass** in Stufe a) Butanol verwendet wird.

15. Mehrstufiges Verfahren nach mindestens einem der vorherigen Ansprüche,
**dadurch gekennzeichnet,**
**dass** die Umsetzung in Stufe a) in Gegenwart von Katalysatoren durchgeführt wird.

16. Mehrstufiges Verfahren nach mindestens einem der vorherigen Ansprüche,
**dadurch gekennzeichnet,**
**dass** die Stufe b) zweistufig durchgeführt wird.

17. Mehrstufiges Verfahren nach Anspruch 16,
**dadurch gekennzeichnet,**
**dass** auf der ersten Stufe die Reaktionsmischung vom Druckniveau der Reaktionsstufe a) auf einen Druck von 1 bis 500 mbar, vorzugsweise 2 bis 150 mbar, entspannt wird.

18. Mehrstufiges Verfahren nach den Ansprüchen 16 oder 17,
**dadurch gekennzeichnet,**
**dass** im zweiten Schritt der flüssige Austrag durch Dünnschichtverdampfung bei 180 °C bis 250 °C, vorzugsweise 200 °C bis 230 °C, und einem Druck von 0,1 mbar bis 20 mbar, vorzugsweise 1 mbar bis 10 mbar, von gegebenenfalls vorhandenem restlichen Alkohol sowie Mittelsiedem wie Dialkylcarbonaten und/oder Carbamidsäurealkylestern befreit wird.

19. Mehrstufiges Verfahren nach den Ansprüchen 16 oder 17,
**dadurch gekennzeichnet,**
**dass** die Brüden der Stufe b) nach weiterer destillativer Reinigung in die Reaktionsstufe a) zugeführt werden.

20. Mehrstufiges Verfahren nach mindestens einem der vorhergehenden Ansprüche,
**dadurch gekennzeichnet,**
**dass** die Trennung in Stufe c), falls angewendet, bei einer Temperatur von 180 bis 270 °C, vorzugsweise 200 bis 250 °C und unter einem Druck von 0,01 bis 10 mbar, vorzugsweise 0,02 bis 5 mbar durchgeführt wird.

21. Mehrstufiges Verfahren nach mindestens einem der vorhergehenden Ansprüche,
**dadurch gekennzeichnet,**
**dass** die Stufe c), falls angewendet, mit Hilfe eines Dünnschicht- oder Kurzwegverdampfers durchgeführt wird.

22. Mehrstufiges Verfahren nach mindestens einem der vorhergehenden Ansprüche,
**dadurch gekennzeichnet,**
**dass** die Nebenprodukte aus Stufe c), falls angewendet, ausgeschleust und verworfen werden.

23. Mehrstufiges Verfahren nach mindestens einem der vorhergehenden Ansprüche,
**dadurch gekennzeichnet,**
**dass** der Stoffstrom in Stufe c), falls angewendet, so verarbeitet wird, dass dieser vor seiner destillativen Aufreinigung in zwei Teilströmen aufgeteilt wird, von denen ein Teilstrom direkt der Spaltreaktion (Stufe d) zugeführt wird.

24. Mehrstufiges Verfahren nach mindestens einem der vorhergehenden Ansprüche,
**dadurch gekennzeichnet,**
**dass** die thermisch induzierte Diurethanspaltung der Stufe d) in Röhrenöfen oder vorzugsweise Verdampfern, wie Fallfilm-, Dünnschicht- oder Bulkverdampfern, ausgewählt aus Robertverdampfern, Herbertverdampfern, caddle-typ-Verdampfern, Oskarverdampfern und Heizkerzenverdampfern durchgeführt wird

25. Mehrstufiges Verfahren nach mindestens einem der vorhergehenden Ansprüche,
**dadurch gekennzeichnet,**
**dass** die Stufe d) in einer kombinierten Spalt- und Rektifizierkolonne durchgeführt wird.

26. Mehrstufiges Verfahren nach mindestens einem der vorhergehenden Ansprüche,
**dadurch gekennzeichnet,**
**dass** in Stufe d) bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch gespalten wird.

27. Mehrstufiges Verfahren nach mindestens einem der vorhergehenden Ansprüche,
**dadurch gekennzeichnet,**
**dass** in Stufe d) lösemittelfrei in flüssiger Phase gespalten wird

28. Mehrstufiges Verfahren nach mindestens einem der vorhergehenden Ansprüche,
**dadurch gekennzeichnet,**
**dass** die Stufe d) in Gegenwart von Katalysatoren durchgeführt wird.

29. Mehrstufiges Verfahren nach mindestens einem der vorhergehenden Ansprüche,
**dadurch gekennzeichnet,**
**dass** in Stufe d) der Umsatz von Diurethan zu Diisocyanat in Abhängigkeit vom verwendeten Diurethan frei gewählt wird, bevorzugt in einem Bereich von 10 bis 95 Gew.-%, vorzugsweise 35 bis 85 Gew.-% der zugeführten Diurethanmenge (Feed) liegt.

30. Mehrstufiges Verfahren nach mindestens einem der vorhergehenden Ansprüche,
**dadurch gekennzeichnet,**
**dass** in Stufe d) ein Teil der Reaktionsmischung, der nicht umgesetzte Diurethane, hochsiedende Nebenprodukte und andere wieder verwertbare und unverwertbare Nebenprodukte enthält, kontinuierlich ausgeschleust wird.

31. Mehrstufiges Verfahren nach Anspruch 30,
**dadurch gekennzeichnet,**
**dass** die Menge der Ausschleusung 10 bis 60 Gew.-%, vorzugsweise 15 bis 45 Gew.-%, bezogen auf den Feed, beträgt.

32. Mehrstufiges Verfahren nach mindestens einem der vorhergehenden Ansprüche,
**dadurch gekennzeichnet,**
**dass** die Stufe e) in einer kombinierten Spalt- und Rektifizierkolonne durchgeführt wird.

33. Mehrstufiges Verfahren nach mindestens einem der vorhergehenden Ansprüche,
**dadurch gekennzeichnet,**
**dass** bei Temperaturen von 95 bis 260 °C, vorzugsweise 110 bis 245 °C und einem Druck von 0,5 bis 250 mbar, vorzugsweise 1 bis 200 mbar verfahren wird.

34. Mehrstufiges Verfahren nach mindestens einem der vorherigen Ansprüchen,
**dadurch gekennzeichnet,**
**dass** in Stufe f) die aus Stufe e) erhaltene rohe Fraktion, bestehend aus cycloaliphatischen Diisocyanat, partiell gespaltenem cycloaliphatischen Diurethan und gegebenenfalls geringen Anteilen an cyclialiphatischen Diurethan, durch Destillation bei einer Temperatur von 95 bis 260 °C, vorzugsweise 110 bis 245 °C und unter einem Druck von 0,5 bis 150 mbar, vorzugsweise 1 bis 75 mbar gereinigt wird.

35. Mehrstufiges Verfahren nach Anspruch 34,
**dadurch gekennzeichnet,**
**dass** die in Stufe f) anfallende Fraktion als Reinprodukt isoliert oder in Stufe g) zurückgeführt wird.

36. Mehrstufiges Verfahren nach mindestens einem der vorhergehenden Ansprüche,
**dadurch gekennzeichnet,**
**dass** in Stufe h) bei einer Temperatur von 180 bis 270 °C, vorzugsweise 200 bis 250 °C und unter Druck von 0,01 bis 10 mbar, vorzugsweise 0,02 bis 5 mbar, verfahren wird.

37. Mehrstufiges Verfahren nach mindestens einem der vorhergehenden Ansprüche,
**dadurch gekennzeichnet,**
**dass** in Stufe h) destillativ mit Hilfe eines Dünnschicht- oder Kurzwegverdampfers verfahren wird.

38. Mehrstufiges Verfahren nach mindestens einem der vorhergehenden Ansprüche,
**dadurch gekennzeichnet,**
**dass** die Stufe h) durch Extraktion erfolgt.

39. Mehrstufiges Verfahren nach mindestens einem der vorhergehenden Ansprüche,
**dadurch gekennzeichnet,**
**dass** der Reurethanisatstrom aus g) vor der destillativen Aufreinigung optional in zwei Teilströme aufgeteilt wird, von denen einer direkt der Reinigungsstufe b) zugeführt wird.

40. Mehrstufiges Verfahren nach Anspruch 39,
**dadurch gekennzeichnet,**
**dass** die Aufteilung der beiden Teilströme im Verhältnis 99 : 1 bis 1 : 99, bevorzugt 95 : 5 bis 5 : 95 erfolgt.

41. Mehrstufiges Verfahren nach mindestens einem der vorhergehenden Ansprüche,
**dadurch gekennzeichnet,**
**dass** die Stufe g) in einer kontinuierlichen Kesselkaskade oder in einem Rohrreaktor durchgeführt wird.

42. Mehrstufiges Verfahren nach mindestens einem der vorhergehenden Ansprüche,
**dadurch gekennzeichnet,**
**dass** die Umsetzung in Stufe g) in Gegenwart von Katalysatoren aus der Gruppe der Sn-und/oder Zn-Carboxylate oder -Halogenide, der tert. Amine, der Cu(I)- und/oder der Fe(III)-Halogeniden erfolgt.

43. Mehrstufiges Verfahren nach mindestens einem der vorhergehenden Ansprüche,
**dadurch gekennzeichnet,**
**dass** in Stufe i) die Rückführung in die Urethanisierungsstufe g) erfolgt.

44. Mehrstufiges Verfahren nach mindestens einem der vorhergehenden Ansprüche,
**dadurch gekennzeichnet,**
**dass** in Stufe i) die Menge der Ausschleusung 0,1 bis 50 Gew.-%, vorzugsweise 0,2 bis 25 Gew.-% des Zulaufs an rohem Polyisocyanat in die Reindestillationsstufe beträgt.

45. Mehrstufiges Verfahren nach mindestens einem der vorhergehenden Ansprüche,
**dadurch gekennzeichnet,**
**dass** in Stufe j) die Menge der pro Zeiteinheit abgeführten Kopffraktion 0,1 bis 3 Gew.-%, vorzugsweise 0,3 bis 1 Gew.- des Zulaufs an rohem Diisocyanat in die Reindestillation beträgt.

46. Mehrstufiges Verfahren nach mindestens einem der vorhergehenden Ansprüche,
**dadurch gekennzeichnet,**
**dass** der zur Hochsiederabtrennung h) führende Reurethanisatstrom aus g) zunächst partiell oder vollständig von überschüssigem Alkohol befreit und der abgetrennte Alkohol wahlweise in Stufe a) oder b) zurückgeführt wird.

47. Mehrstufiges Verfahren nach mindestens einem der vorhergehenden Ansprüche,
**dadurch gekennzeichnet,**
**dass** der Wertstoffstrom aus der Hochsiederabtrennung h) in Stufe a), b) oder d) zurückgeführt wird.

48. Mehrstufiges Verfahren nach mindestens einem der vorhergehenden Ansprüche,
**dadurch gekennzeichnet,**
**dass** 1,4-Diisocyanatocyclohexan, 4,4'-Methylendicyclohexyldiisocyanat, 2,2'-Methylendicyclohexyldiisocyanat, 2,4'-Methylendicyclohexyldiisocyanat oder auch beliebige Mischungen mindestens zweier isomerer Methylendicyclohexyldiisocyanate hergestellt werden.

49. Mehrstufiges Verfahren nach mindestens einem der vorherigen Ansprüche,
**dadurch gekennzeichnet,**
**dass** Diamine ausgewählt aus 1,3- und 1,4-Diaminomethylcyclohexan, Hexandiamin-1,6, 2,2,4- bzw. 2,4,4-Trimethylhexanamin-1,6 und 3-Aminomethyl-3,5,5-trimethylcyclohexylamin eingesetzt werden.

## Claims

1. Multistage process for continuously preparing cycloaliphatic diisocyanates, by reacting cycloaliphatic diamines with carbonic acid derivatives and alcohols to give cycloaliphatic diurethanes and subsequently thermally cleaving the diurethanes to give cycloaliphatic diisocyanates,
which comprises
freeing the cycloaliphatic diurethanes of low and medium boilers after they have been synthesized by reacting cycloaliphatic diamines with alcohol and urea and/or urea derivatives, thermally cleaving the cycloaliphatic diurethanes purified in this way to release the desired diisocyanate, continuously discharging a portion of the cleavage residue from the cleavage apparatus and reurethanizing it with alcohol, and then removing the high boiler components and recycling the stream purified in this way into the process.

2. Multistage process according to Claim 1 for continuously preparing cycloaliphatic diisocyanates of the formula (I)
OCN-R-NCO
where R is a bivalent cycloaliphatic hydrocarbon radical having from 4 to 18, preferably from 5 to 15, carbon atoms, with the proviso that the two nitrogen atoms are bonded directly to a hydrocarbon cycle and at least 3 carbon atoms are disposed between them, by reacting cycloaliphatic diamines with urea and/or urea derivatives and alcohols to give cycloaliphatic diurethanes and thermally cleaving them, wherein
a) cycloaliphatic diamines of the formula (II)
H₂N-R-NH₂
where R is a bivalent cycloaliphatic hydrocarbon radical having from 4 to 18, preferably from 5 to 15, carbon atoms, with the proviso that the two nitrogen atoms are bonded directly to a hydrocarbon cycle and at least 3 carbon atoms being disposed between them, are reacted with urea and/or urea derivatives and alcohols of the formula (III)
R¹-OH
where R¹ is a radical as remains after removal of the hydroxyl group from a primary or secondary (cyclo)aliphatic alcohol having from 3 to 8 carbon atoms, in the absence or presence of dialkyl carbonates, alkyl carbamates or mixtures of dialkyl carbonates and carbamic esters and in the absence or presence of catalysts, to give cycloaliphatic diurethanes, and the ammonia formed is simultaneously removed;
b) the alcohol, the dialkyl carbonates and/or alkyl carbamates are removed from the resulting reaction mixture, and the alcohol and optionally also the dialkyl carbonates and/or alkyl carbamates are recycled into the reaction stage a);
c) a removal of any high-boiling residues present in the resulting reaction mixture is fully or partially dispensed with;
d) the reaction mixture comprising the diurethanes purified by steps b) and optionally c) is continuously and thermally cleaved without solvent in the presence of a catalyst, at temperatures of from 180 to 280°C, preferably from 200 to 260°C, and under a pressure of from 0.1 to 200 mbar, preferably from 0.2 to 100 mbar, in such a way that a portion of the reaction mixture of from 10 to 60% by weight based on the feed, preferably from 15 to 45% by weight based on the feed, is constantly discharged;
e) the cleavage products are separated by rectification into crude cycloaliphatic diisocyanate and alcohol;
f) the crude cycloaliphatic diisocyanate, purified by distillation, and the pure product fraction are isolated;
g) the bottoms discharge from d) is reacted partially or fully with the alcohol from e), in the presence or absence of catalysts, within from 1 to 150 min, preferably from 3 to 60 min, at temperatures of from 20 to 200°C, preferably from 50 to 170°C, and at a pressure of from 0.5 to 20 bar, preferably from 1 to 15 bar, the molar ratio of NCO groups to OH groups being up to 1:100, preferably 1:20 and more preferably 1:10;
h) the reurethanized stream from g) is separated into a material-of-value stream and a waste stream and the waste stream rich in high boiler components is discharged from the process and discarded;
i) a portion of the bottoms fraction of the purification by distillation f) is continuously discharged and conducted into the cleavage reaction d) or into the urethanization stage g);
j) optionally, the top fraction obtained in the purification by distillation f) of the crude cycloaliphatic diisocyanate is likewise recycled into the urethanization stage g);
k) the material-of-value stream from h) is recycled into stage a), b) or d).

3. Multistage process according to Claim 1 or 2
**characterized in that**
the cycloaliphatic diamine used is 4,4'-dicyclohexylmethanediamine, 2,4-dicyclohexylmethanediamine and 2,2'-dicyclohexylmethanediamine, and also any mixtures of at least two of these isomers.

4. Multistage process according to Claim 1 or 2,
**characterized in that**
the cycloaliphatic diamine used is 4,4'-dicyclohexylmethanediamine and/or isomeric cycloaliphatic diamines.

5. Multistage process according to Claim 1 or 2,
**characterized in that**
the cycloaliphatic diamine used is 1,4-diaminocyclohexane.

6. Multistage process according to at least one of the preceding claims,
**characterized in that**
stage a) is carried out continuously in a distillation reactor or in a stirred tank battery.

7. Multistage process according to at least one of the preceding claims,
**characterized in that**
the reaction in stage a) is effected in a molar ratio of diamine : urea : alcohol of from 1:2.01:4.0 to 1:2.2:10.

8. Multistage process according to at least one of the preceding claims,
**characterized in that** the residence time of the reactants in stage a) is from 2 to 10 hours, preferably from 4 to 9 hours.

9. Multistage process according to at least one of the preceding claims,
**characterized in that**
stage a) is carried out in a reactor at from 140 to 270°C and a pressure of from 2 to 80 bar.

10. Multistage process according to at least one of the preceding claims, **characterized in that** the reaction in stage a) is carried out at reaction temperatures of from 160 to 250°C and a pressure of from 7 to 15 bar.

11. Multistage process according to at least one of the preceding claims,
**characterized in that**
stage a) is carried out in the pressure distillation reactor.

12. Multistage process according to at least one of the preceding claims,
**characterized in that**, in stage a), the reactants are supplied continuously to the uppermost tray and the ammonia released is driven out supported by alcohol vapors which are introduced into the bottom of the distillation reactor.

13. Multistage process according to at least one of the preceding claims,
**characterized in that**,
in stage a), alcohols having 1-6 carbon atoms are used.

14. Multistage process according to at least one of the preceding claims,
**characterized in that**,
in stage a), butanol is used.

15. Multistage process according to at least one of the preceding claims,
**characterized in that**
the reaction in stage a) is carried out in the presence of catalysts.

16. Multistage process according to at least one of the preceding claims,
**characterized in that**
stage b) is carried out in two stages.

17. Multistage process according to Claim 16,
**characterized in that**,
at the first stage, the reaction mixture is decompressed from the pressure level of reaction stage a) to a pressure of from 1 to 500 mbar, preferably from 2 to 150 mbar.

18. Multistage process according to Claims 16 or 17,
**characterized in that**,
in the second step, the liquid effluent is freed of any residual alcohol present and also of medium boilers such as dialkyl carbonates and/or alkyl carbamates by thin-film evaporation at from 180°C to 250°C, preferably from 200°C to 230°C, and a pressure of from 0.1 mbar to 20 mbar, preferably from 1 mbar to 10 mbar.

19. Multistage process according to Claims 16 or 17,
**characterized in that**
the vapors of stage b) are fed, after further distillative purification, into reaction stage a).

20. Process according to at least one of the preceding claims,
**characterized in that**
the separation in stage c), if employed, is carried out at a temperature of from 180 to 270°C, preferably from 200 to 250°C, and under a pressure of from 0.01 to 10 mbar, preferably from 0.02 to 5 mbar.

21. Multistage process according to at least one of the preceding claims,
**characterized in that**
stage c), if employed, is carried out with the aid of a thin-film or short-path evaporator.

22. Multistage process according to at least one of the preceding claims,
**characterized in that**
the by-products from stage c), if employed, are discharged and discarded.

23. Multistage process according to at least one of the preceding claims,
**characterized in that**
the stream in stage c), if this is employed, is processed in such a way that it is divided before its distillative purification into two substreams of which one substream is fed directly to the cleavage reaction (step d).

24. Multistage process according to at least one of the preceding claims,
**characterized in that**
the thermally induced diurethane cleavage of stage d) is carried out in tubular furnaces or preferably evaporators such as falling-film, thin-film or bulk evaporators, selected from Robert evaporators, Herbert evaporators, Caddle-type evaporators, Oskar evaporators and heating cartridge evaporators.

25. Multistage process according to at least one of the preceding claims,
**characterized in that**
the stage d) is carried out in a combined cleavage and rectification column.

26. Multistage process according to at least one of the preceding claims,
**characterized in that**,
in stage d), thermal cleavage is effected continuously at temperatures of from 180 to 280°C, preferably from 200 to 260°C, and under a pressure of from 0.1 to 200 mbar, preferably from 0.2 to 100 mbar.

27. Multistage process according to at least one of the preceding claims,
**characterized in that**,
in stage d), cleavage is effected without solvent in the liquid phase.

28. Multistage process according to at least one of the preceding claims,
**characterized in that**
stage d) is carried out in the presence of catalysts.

29. Multistage process according to at least one of the preceding claims,
**characterized in that**,
in stage d), the conversion of diurethane to diisocyanate is selected freely depending on the diurethane used, preferably within the range of from 10 to 95% by weight, preferably from 35 to 85% by weight of the diurethane feed.

30. Multistage process according to at least one of the preceding claims,
**characterized in that**,
in stage d), a portion of the reaction mixture which comprises unconverted diurethanes, high-boiling by-products and other reutilizable and nonutilizable by-products is continuously discharged.

31. Multistage process according to Claim 30,
**characterized in that**
the amount of the discharge is from 10 to 60% by weight, preferably from 15 to 45% by weight, based on the feed.

32. Multistage process of at least one of the preceding claims,
**characterized in that**
stage e) is carried out in a combined cleavage and rectification column.

33. Multistage process according to at least one of the preceding claims,
**characterized in that**
operation is effected at temperatures of from 95 to 260°C, preferably from 110 to 245°C, and a pressure of from 0.5 to 250 mbar, preferably from 1 to 200 mbar.

34. Multistage process according to at least one of the preceding claims,
**characterized in that**,
in stage f), the crude fraction obtained from stage e), consisting of cycloaliphatic diisocyanate, partially cleaved cycloaliphatic diurethane and in some cases small fractions of cycloaliphatic diurethane, is purified by distillation at a temperature of from 95 to 260°C, preferably from 110 to 245°C, and under a pressure of from 0.5 to 150 mbar, preferably from 1 to 75 mbar.

35. Multistage process according to Claim 34,
**characterized in that**
the fraction obtained in stage f) is isolated as a pure product or recycled into stage g).

36. Multistage process according to at least one of the preceding claims,
**characterized in that**,
in stage h), operation is effected at a temperature of from 180 to 270°C, preferably from 200 to 250°C, and under a pressure of from 0.01 to 10 mbar, preferably from 0.02 to 5 mbar.

37. Multistage process according to at least one of the preceding claims,
**characterized in that**
in stage h), operation is effected distillatively with the aid of a thin-film or short-path evaporator.

38. Multistage process according to at least one of the preceding claims,
**characterized in that**
stage h) is effected by extraction.

39. Multistage process according to at least one of the preceeding claims, **characterized in that**
the reurethanized stream from g), before the distillative purification, is optionally divided into two substreams, of which one is fed directly to the purificaction stage b).

40. Multistage process according to Claim 39,
**characterized in that**
the two substreams are divided in a ratio of from 99:1 to 1:99, preferably from 95:5 to 5:95.

41. Multistage process according to at least one of the preceding claims,
**characterized in that**
stage i) is carried out in a continuous tank battery or in a tubular reactor.

42. Multistage process according to at least one of the preceding claims,
**characterized in that**
the reaction in stage g) is effected in the presence of catalysts from the group of tin or zinc carboxylates or halides, tertiary amines and Cu(I) and/or Fe (III) halides.

43. Multistage process according to at least one of the preceding claims,
**characterized in that**,
in stage i), the recycling is effected into the urethanization stage g).

44. Multistage process according to at least one of the preceding claims,
**characterized in that**,
in stage j), the amount of the discharge is from 0.1 to 50% by weight, preferably from 0.2 to 25% by weight, of the feed of crude polyisocyanate into the purifying distillation stage.

45. Multistage process according to at least one of the preceding claims,
**characterized in that**
the amount of top fraction removed per unit time in step j) is from 0.1 to 3% by weight, preferably from 0.3 to 1% by weight, of the feed of crude diisocyanate into the purifying distillation.

46. Multistage process according to at least one of the preceding claims,
**characterized in that**
the reurethanized stream from g) leading to the high boiler removal h) is initially freed partially or fully of excess alcohol and the alcohol removed is recycled as desired into stage a) or b).

47. Multistage process according to at least one of the preceding claims,
**characterized in that**
the material-of-value stream from the high boiler removal h) is recycled into stage a), b) or d).

48. Multistage process according to at least one of the preceding claims,
**characterized in that**
1,4-diisocyanatocyclohexane, 4,4'-dicyclohexylmethane diisocyanate, 2,2'-dicyclohexylmethane diisocyanate, 2,4-dicyclohexylmethane diisocyanate or else any mixtures of at least two isomeric dicyclohexylmethane diisocyanates are prepared.

49. Multistage process according to at least one of the preceding claims,
**characterized in that**
diamines selected from 1,3- and 1,4-diaminomethylcyclohexane, hexane-1,6-diamine, 2,2,4- and 2,4,4-trimethylhexan-1,6-amine and 3-aminomethyl-3,5,5-trimethylcyclohexylamine are used.

## Revendications

1. Procédé à plusieurs étapes pour la préparation en continu de diisocyanates cycloaliphatiques par transformation de diamines cycloaliphatiques avec des dérivés de l'acide carbonique et des alcools en diuréthanes cycloaliphatiques, suivie d'une dissociation thermique des diuréthanes en diisocyanates cycloaliphatiques, **caractérisé en ce que** les diuréthanes cycloaliphatiques sont libérés, après leur synthèse par transformation de diamines cycloaliphatiques avec un alcool et de l'urée et/ou des dérivés d'urée, de la fraction à bas point d'ébullition et à point d'ébullition moyen, les diuréthanes cycloaliphatiques ainsi purifiés sont dissociés thermiquement avec libération du diisocyanate souhaité, une partie du fond de la dissociation est soutirée en continu de l'appareillage de dissociation et réuréthanisé par de l'alcool, puis les composants à point d'ébullition élevé sont séparés et le flux de substances ainsi purifié est recyclé dans le procédé.

2. Procédé en plusieurs étapes selon la revendication 1 pour la préparation en continu de diisocyanates cycloaliphatiques de formule (I)
OCN-R-NCO
dans laquelle R représente un radical hydrocarboné divalent cycloaliphatique comprenant 4 à 18, de préférence 5 à 15 atomes de carbone, sous réserve que les deux atomes d'azote soient liés directement à un cycle hydrocarboné et qu'au moins 3 atomes de carbone soient interposés entre eux, par transformation de diamines cycloaliphatiques avec de l'urée et/ou des dérivés d'urée et des alcools en diuréthanes cycloaliphatiques et leur dissociation thermique, où
a) on transforme des diamines cycloaliphatiques de la formule (II)
H₂N-R-NH₂
dans laquelle R représente un radical hydrocarboné divalent cycloaliphatique comprenant 4 à 18, de préférence 5 à 15 atomes de carbone, sous réserve que les deux atomes d'azote soient liés directement à un cycle hydrocarboné et qu'au moins 3 atomes de carbone soient interposés entre eux, avec de l'urée et/ou des dérivés d'urée et des alcools de formule (III)
R¹-OH
dans laquelle R¹ représente un radical tel qu'il reste après l'élimination du groupe hydroxyle d'un alcool (cyclo) aliphatique primaire ou secondaire comprenant 3 à 8 atomes de carbone,
en l'absence ou en présence de carbonates de dialkyle, d'esters alkyliques de l'acide carbamique ou de mélanges de carbonates de dialkyle et d'esters de l'acide carbamique et en l'absence ou en présence de catalyseurs, en diuréthanes cycloaliphatiques et on sépare simultanément l'ammoniac qui se forme ;
b) on sépare l'alcool, les carbonates de dialkyle et/ou les esters alkyliques de l'acide carbamique du mélange réactionnel obtenu et on recycle l'alcool ainsi qu'éventuellement aussi les carbonates de dialkyle et/ou les esters alkyliques de l'acide carbamique dans l'étape de réaction a) ;
c) on renonce totalement ou partiellement à une séparation des résidus à haut point d'ébullition le cas échéant contenus dans le mélange réactionnel obtenu ;
d) on dissocie thermiquement le mélange réactionnel contenant les diuréthanes purifiés dans les étapes b) et éventuellement c) en présence d'un catalyseur, en continu et sans solvant à des températures de 180 à 280 °C, de préférence de 200 à 260°C, et sous une pression de 0,1 à 200 mbars, de préférence de 0,2 à 100 mbars, de telle sorte qu'une partie du mélange réactionnel de 10 à 60% en poids par rapport à l'alimentation, de préférence de 15 à 45% en poids par rapport à l'alimentation, est constamment soutirée ;
e) les produits de dissociation sont séparés par rectification en un diisocyanate cycloaliphatique brut et en alcool ;
f) le diisocyanate cycloaliphatique brut est purifié par distillation et la fraction de produit pur est isolée ;
g) le produit soutiré du fond de d) est transformé, partiellement ou complètement, avec l'alcool de e) en présence ou en l'absence de catalyseurs en l'espace de 1 à 150 min, de préférence de 3 à 60 min, à des températures de 20 à 200°C, de préférence de 50 à 170°C et à une pression de 0,5 à 20 bars, de préférence de 1 à 15 bars, le rapport molaire des groupes NCO aux groupes OH valant jusqu'à 1:100, de préférence 1:20 et de façon particulièrement préférée 1:10 ;
h) le flux de ré-uréthanisation de g) est séparé en un flux de produit de valeur et en un flux de déchet et le flux de déchet riche en composants à haut point d'ébullition est soutiré du procédé et est rejeté ;
i) une partie de la fraction du fond de la distillation pure f) est soutirée en continu et guidée dans la réaction de dissociation d) ou dans l'étape d'uréthanisation g) ;
j) la fraction de tête du diisocyanate cycloaliphatique brut se formant lors de la distillation pure f) est éventuellement également recyclée dans l'étape d'uréthanisation g) ;
k) le flux de produit de valeur de h) est recyclé dans l'étape a), b) ou d).

3. Procédé à plusieurs étapes selon la revendication 1 ou 2, **caractérisé en ce qu'**on utilise, comme diamine cycloaliphatique, la 4,4'-méthylènedicyclohexyldiamine, la 2,4-méthylènedicyclohexyldiamine et la 2,2'-méthylènedicyclohexyldiamine ainsi qu'également des mélanges quelconques d'au moins deux de ces isomères.

4. Procédé à plusieurs étapes selon la revendication 1 ou 2, **caractérisé en ce qu'**on utilise comme diamine cycloaliphatique la 4,4'-méthylènedicyclohexyldiamine et/ou des diamines cycloaliphatiques isomères.

5. Procédé à plusieurs étapes selon la revendication 1 ou 2, **caractérisé en ce qu'**on utilise comme diamine cycloaliphatique du 1,4-diaminocyclohexane.

6. Procédé à plusieurs étapes selon au moins l'une quelconque des revendications précédentes, **caractérisé en ce que** l'étape a) est réalisée en continu dans un réacteur de distillation ou dans une cascade de cuves agitées.

7. Procédé à plusieurs étapes selon au moins l'une quelconque des revendications précédentes, **caractérisé en ce que** la transformation dans l'étape a) a lieu à un rapport molaire de diamine:urée:alcool de 1:2,01:4,0 à 1:2,2:10.

8. Procédé à plusieurs étapes selon au moins l'une quelconque des revendications précédentes, la durée de séjour des produits de départ dans l'étape a) étant de 2 à 20, de préférence de 4 à 9 heures.

9. Procédé à plusieurs étapes selon au moins l'une quelconque des revendications précédentes, **caractérisé en ce que** l'étape a) est réalisée dans un réacteur à 140 jusqu'à 270°C et une pression de 2 à 80 bars.

10. Procédé à plusieurs étapes selon au moins l'une quelconque des revendications précédentes, **caractérisé en ce qu'**on transforme, dans l'étape a), à des températures de réaction de 160 à 250°C et à une pression de 7 à 15 bars.

11. Procédé à plusieurs étapes selon au moins l'une quelconque des revendications précédentes, **caractérisé en ce que** l'étape a) est réalisée dans un réacteur de distillation sous pression.

12. Procédé à plusieurs étapes selon au moins l'une quelconque des revendications précédentes, dans lequel, dans l'étape a), les produits de départ sont introduits en continu sur le plateau supérieur et l'ammoniac libéré est évacué de manière soutenue par les vapeurs d'alcool, qui sont introduites dans le fond du réacteur de distillation.

13. Procédé à plusieurs étapes selon au moins l'une quelconque des revendications précédentes, **caractérisé en ce qu'**on utilise, dans l'étape a), des alcools comprenant 1-6 atomes de carbone.

14. Procédé à plusieurs étapes selon au moins l'une quelconque des revendications précédentes, **caractérisé en ce qu'**on utilise du butanol dans l'étape a).

15. Procédé à plusieurs étapes selon au moins l'une quelconque des revendications précédentes, **caractérisé en ce que** la transformation dans l'étape a) est réalisée en présence de catalyseurs.

16. Procédé à plusieurs étapes selon au moins l'une quelconque des revendications précédentes, **caractérisé en ce que** l'étape b) est réalisée en deux étapes.

17. Procédé à plusieurs étapes selon la revendication 16, **caractérisé en ce qu'**on détend, après la première étape, le mélange réactionnel du niveau de pression de l'étape de réaction a) à une pression de 1 à 500 mbars, de préférence de 2 à 150 mbars.

18. Procédé à plusieurs étapes selon les revendications 16 ou 17, **caractérisé en ce que** dans la deuxième étape, le produit évacué liquide est libéré par évaporation en couche mince à 180°C jusqu'à 250°C, de préférence à 200°C jusqu'à 230°C, et à une pression de 0,1 mbar à 20 mbars, de préférence de 1 mbar à 10 mbars, de l'alcool résiduel le cas échéant présent ainsi que de la fraction à point d'ébullition moyen, telle que les carbonates de dialkyle et/ou les esters alkyliques de l'acide carbamique.

19. Procédé à plusieurs étapes selon les revendications 16 ou 17, **caractérisé en ce que** les vapeurs de l'étape b) sont introduites dans l'étape de procédé a) après une nouvelle purification par distillation.

20. Procédé à plusieurs étapes selon au moins l'une quelconque des revendications, **caractérisé en ce que** la séparation dans l'étape c), si elle est appliquée, est réalisée à une température de 180 à 270°C, de préférence de 200 à 250°C et sous une pression de 0,01 à 10 mbars, de préférence de 0,02 à 5 mbars.

21. Procédé à plusieurs étapes selon au moins l'une quelconque des revendications, **caractérisé en ce que** l'étape c), si elle est appliquée, est réalisée à l'aide d'un évaporateur à couche mince ou instantané.

22. Procédé à plusieurs étapes selon au moins l'une quelconque des revendications, **caractérisé en ce que** les produits secondaires de l'étape c), si elle est appliquée, sont soutirés et rejetés.

23. Procédé à plusieurs étapes selon au moins l'une quelconque des revendications, **caractérisé en ce que** le flux de substances dans l'étape c), si elle est appliquée, est transformé de manière telle qu'il est réparti, avant sa purification par distillation, en deux flux partiels, dont un flux partiel est alimenté directement dans la réaction de dissociation (étape d).

24. Procédé à plusieurs étapes selon au moins l'une quelconque des revendications, **caractérisé en ce que** la dissociation de diuréthane induite thermiquement de l'étape d) est réalisée dans des fours tubulaires ou de préférence des évaporateurs, tels que des évaporateurs à film tombant, à couche mince ou en vrac, choisis parmi les évaporateurs de Robert, de Herbert, de type caddle, d'Oskar et à bougie chauffante.

25. Procédé à plusieurs étapes selon au moins l'une quelconque des revendications, **caractérisé en ce que** l'étape d) est réalisée dans une colonne combinée de dissociation et de rectification.

26. Procédé à plusieurs étapes selon au moins l'une quelconque des revendications, **caractérisé en ce qu'**on dissocie thermiquement en continu dans l'étape d) à des températures de 180 à 280°C, de préférence de 200 à 260°C, et à une pression de 0,1 à 200 mbars, de préférence de 0,2 à 100 mbars.

27. Procédé à plusieurs étapes selon au moins l'une quelconque des revendications, **caractérisé en ce que** qu'on dissocie en phase liquide sans solvant dans l'étape d).

28. Procédé à plusieurs étapes selon au moins l'une quelconque des revendications, **caractérisé en ce que** l'étape d) est réalisée en présence de catalyseurs.

29. Procédé à plusieurs étapes selon au moins l'une quelconque des revendications, **caractérisé en ce que** dans l'étape d), la conversion de diuréthane en diisocyanate est choisie librement en fonction du diuréthane utilisé, de préférence dans une plage de 10 à 95% en poids, de préférence de 35 à 85% en poids de la quantité de diuréthane alimentée (alimentation).

30. Procédé à plusieurs étapes selon au moins l'une quelconque des revendications, **caractérisé en ce que** dans l'étape d), une partie du mélange réactionnel, qui contient des diuréthanes non transformés, des produits secondaires à point d'ébullition élevé et d'autres produits secondaires valorisables et non valorisables, est soutirée en continu.

31. Procédé à plusieurs étapes selon la revendication 30, **caractérisé en ce que** la quantité soutirée est de 10 à 60% en poids, de préférence de 15 à 45% en poids, par rapport à l'alimentation.

32. Procédé à plusieurs étapes selon au moins l'une quelconque des revendications, **caractérisé en ce que** l'étape e) est réalisée dans une colonne combinée de dissociation et de rectification.

33. Procédé à plusieurs étapes selon au moins l'une quelconque des revendications, **caractérisé en ce qu'**on procède à des températures de 95 à 260°C, de préférence de 110 à 245°C et à une pression de 0,5 à 250 mbars, de préférence de 1 à 200 mbars.

34. Procédé à plusieurs étapes selon au moins l'une quelconque des revendications précédentes, **caractérisé en ce que** dans l'étape f), la fraction brute obtenue dans l'étape e), constituée par du diisocyanate cycloaliphatique, du diuréthane cycloaliphatique partiellement dissocié et le cas échéant des proportions plus faibles de diuréthane cycloaliphatique, est purifiée par distillation à une température de 95 à 260°C, de préférence de 110 à 245°C et à une pression de 0,5 à 150 mbars, de préférence de 1 à 75 mbars.

35. Procédé à plusieurs étapes selon la revendication 34, **caractérisé en ce que** la fraction produite dans l'étape f) est isolée en tant que produit pur ou recyclée dans l'étape g).

36. Procédé à plusieurs étapes selon au moins l'une quelconque des revendications, **caractérisé en ce qu'**on procède dans l'étape h) à une température de 180 à 270°C, de préférence de 200 à 250°C et à une pression de 0,01 à 10 mbars, de préférence de 0,02 à 5 mbars.

37. Procédé à plusieurs étapes selon au moins l'une quelconque des revendications, **caractérisé en ce qu'**on procède dans l'étape h) par distillation à l'aide d'un évaporateur à couche mince ou instantané.

38. Procédé à plusieurs étapes selon au moins l'une quelconque des revendications, **caractérisé en ce que** l'étape h) a lieu par extraction.

39. Procédé à plusieurs étapes selon au moins l'une quelconque des revendications, **caractérisé en ce que** le flux de ré-uréthanisation de g) est réparti, avant la purification par distillation, éventuellement en deux flux partiels, dont l'un est alimenté directement dans l'étape de purification b).

40. Procédé à plusieurs étapes selon la revendication 39, **caractérisé en ce que** la répartition des deux flux partiels a lieu dans un rapport de 99:1 à 1:99, de préférence de 95:5 à 5:95.

41. Procédé à plusieurs étapes selon au moins l'une quelconque des revendications, **caractérisé en ce que** l'étape g) est réalisée dans une cascade continue de cuves ou dans un réacteur tubulaire.

42. Procédé à plusieurs étapes selon au moins l'une quelconque des revendications, **caractérisé en ce que** la transformation dans l'étape g) a lieu en présence de catalyseurs du groupe des carboxylates ou des halogénures de Sn et/ou de Zn, des tert-amines, des halogénures de Cu(I) et/ou de Fe(III).

43. Procédé à plusieurs étapes selon au moins l'une quelconque des revendications, **caractérisé en ce que** dans l'étape i), le recyclage a lieu dans l'étape d'uréthanisation g).

44. Procédé à plusieurs étapes selon au moins l'une quelconque des revendications, **caractérisé en ce que** dans l'étape i), la quantité soutirée représente 0,1 à 50% en poids, de préférence 0,2 à 25% en poids de l'alimentation en polyisocyanate brut dans l'étape de distillation pure.

45. Procédé à plusieurs étapes selon au moins l'une quelconque des revendications, **caractérisé en ce que** dans l'étape j), la quantité de fraction de tête évacuée par unité de temps représente 0,1 à 3% en poids, de préférence 0,3 à 1% en poids de l'alimentation en diisocyanate brut dans la distillation pure.

46. Procédé à plusieurs étapes selon au moins l'une quelconque des revendications, **caractérisé en ce que** le flux de ré-uréthanisation de g) dirigé vers la séparation de la fraction à point d'ébullition élevé h) est d'abord libéré partiellement ou complètement de l'alcool en excès et l'alcool séparé est au choix recyclé dans l'étape a) ou l'étape b).

47. Procédé à plusieurs étapes selon au moins l'une quelconque des revendications, **caractérisé en ce que** le flux de produit de valeur de la séparation de la fraction à point d'ébullition élevé h) est recyclé dans l'étape a), b) ou d).

48. Procédé à plusieurs étapes selon au moins l'une quelconque des revendications, **caractérisé en ce qu'**on prépare du 1,4-diisocyanatocyclohexane, du diisocyanate de 4,4'-méthylènedicyclohexyle, du diisocyanate de 2,2'-méthylènedicyclohexyle, du diisocyanate de 2,4'-méthylènedicyclohexyle ou également des mélanges quelconques d'au moins deux diisocyanates de méthylènedicyclohexyle isomères.

49. Procédé à plusieurs étapes selon au moins l'une quelconque des revendications précédentes, **caractérisé en ce qu'**on utilise des diamines choisies parmi le 1,3-diaminométhylcyclohexane et le 1,4-diaminométhylcyclohexane, l'hexanediamine-1,6, la 2,2,4-triméthylhexanamine-1,6 ou, selon le cas la 2,4,4-triméthylhexanamine-1,6 et la 3-aminométhyl-3,5,5-triméthylcyclohexylamine.
